# NEW EUROPEAN PATENT SPECIFICATION

(11) **EP 1 720 974 B2**
(45) Date of publication and mention of the opposition decision: **22.12.2021**
(45) Mention of the grant of the patent: 28.04.2010
(21) Application number: 05706788.6
(22) Date of filing: 24.02.2005
(51) Int. Cl.: C12N 1/00, C12N 1/04, A23K 1/00

(54) **FROZEN LACTIC ACID BACTERIA CULTURE OF INDIVIDUAL PELLETS**
TIEFGEFRORENE GRANULATBILDENDE MILCHSÄUREBAKTERIENKULTUR
GRAINS CONGELES INDIVIDUELS DE CULTURES DE BACTERIES LACTIQUES

(30) Priority: 24.02.2004 EP 04100714
(43) Date of publication of application: 15.11.2006
(60) Divisional application: 10160850.3
(73) Proprietor: Chr. Hansen A/S, 2970 Horsholm (DK)
(72) Inventor: STAVNSBJERG, Rikke, DK-2860 Søborg (DK); KNAP, Inge, DK-3520 Farum (DK); BISGAARD-FRANTZEN, Hans, DK-2610 Rødovre (DK)
(74) Representative: Uexküll & Stolberg
(86) International application number: PCT/DK2005/000126
(87) International publication number: WO 2005/080548

(56) References cited:
- WO-A-00/39281
- US-A- 4 140 800

## Description

### FIELD OF THE INVENTION:

The present invention relates to a pellet-frozen lactic acid bacteria (LAB) culture in a commercially relevant package that has a weight of at least 50 g frozen material, wherein the frozen material is present in the form of individual pellets characterized by the fact that when stored at a temperature below the initial melting temperature (Tm') of the culture, e.g. at -46°C, for 7-14 days the individual pellets of the frozen culture are not sticking together and therefore substantially remain as individual pellets.

### BACKGROUND OF THE INVENTION:

Microorganisms are involved in the manufacture of food and feed products including most dairy products. Bacterial cultures, in particular cultures of bacteria that are generally classified as lactic acid bacteria, are essential in the making of all fermented milk products, cheese and butter. Cultures of such bacteria may be referred to as starter cultures and they impart specific features to various dairy products by performing a number of functions.

Dairy starter cultures are generally composed of lactic acid bacteria. In the present context, the expression "lactic acid bacteria" (LAB) designates a group of Gram positive, catalase negative, non-motile, non-sporulating, microaerophilic or anaerobic bacteria which ferment sugars with the production of organic acids, including lactic acid as the predominantly produced acid, formic acid and propionic acid. In the present context lactic acid bacteria comprise of a number of bacterial genera within the phylum *Firmicutes.* The genera *Carnobacterium*, *Enterococcus, Lactobacillus, Lactococcus, Lactosphaera*, *Leuconostoc, Melissococcus, Oencoccus*, *Pediococcus*, *Streptococcus*, *Tetragenococcus*, *Vagococcus* and *Weissella* are recognized as LAB. Also lactic acid-producing Gram-positive bacteria belonging to the phylum *Actinobacteria* such as the genera *Aerococcus*, *Microbacterium* and *Propionibacterium* as well as *Bifidobacterium* are in the present context considered as LAB. The industrially most useful lactic acid bacteria are found among *Lactococcus* species, *Streptococcus*, species, *Enterococcus* species, *Lactobacillus* species, *Leuconostoc* species, *Bifidobacterium* species and *Pediococcus* species. In addition to the their use in the dairy industry lactic acid bacteria cultures also find widely use in the meat processing industry as well as a number of other industries.

Commercial starter cultures may be distributed as frozen cultures. Highly concentrated frozen cultures are commercially very interesting since such cultures can be inoculated directly into the fermentation medium (e.g. milk or meat) without intermediate transfer. In others words, such highly concentrated frozen cultures comprise bacteria in an amount that makes in-house bulk starter cultures at the end-users superfluous. A "bulk starter" is defined herein as a starter culture propagated at the food processing plant for inoculation into the fermentation medium. Highly concentrated cultures may be referred to as direct vat set (DVS)-cultures. In order to comprise sufficient bacteria to be used as a DVS-culture at the end-users, a concentrated frozen culture generally has to have a weight of at least 50 g and a content of viable bacteria of at least 10⁹ colony forming units (CFU) per g.

An important issue in the practical use of frozen cultures is the convenience of the actual handling of the cultures. Whereas cultures frozen "en block" are difficult to handle it has been found that cultures frozen in pellets are very easy to handle both for the producer and the consumer.

Consequently, a thriving market for highly concentrated pellet frozen cultures - so-called frozen direct vat set (F-DVS)-cultures - has formed.

A number of publications concerned with the viability of frozen cultures have occurred.

Chavarri et al. (1988) describes that the viability of a frozen pure *Streptococcus*, *lactis* culture can be improved by addition of 5% lactose or 5% sucrose.

Cárcoba et al. (2000) describes that the viability of a frozen pure *Lactococcus lactis* subsp. *lactis* culture can be improved by addition of different cryoprotective agents such as sugars (lactose, sucrose and trehalose), glutamic acid and gelatin.

US 4.140.800 (Kline), describes that the viability of freeze-dried cultures can be improved by addition of different cryoprotective agents. Also the viability of frozen cultures added lactose, sucrose or maltose are discussed.

WO00/39281 (Kringelum et. al.) describes that the viability of a non-frozen, liquid starter culture can be improved by addition of different cryoprotective agents, and

WO 2004/065584 A1 (Bisgaard-Frantzen) describes that the viability of a highly concentrated frozen starter culture can be improved by addition of different cryoprotective agents.

Only WO 2004/065584 A1 describes pellet-frozen cultures and none of the above-mentioned publications refer to the physical stability of the pellet-frozen cultures during storage.

### SUMMARY OF THE INVENTION:

Commercially, a pellet-frozen lactic acid bacteria (LAB) culture is normally provided in a suitable package (e.g. in a 2 L tetra pack of carton). It is normally stored at a storage temperature of around -46°C and the frozen material is present in the form of individual pellets of a relatively small weight.

Prior to the present invention, the present inventors believed that there were no significant problems with respect to storage of such commercial relevant pellet-frozen lactic acid bacteria (LAB) cultures.

However, based on different studies the present inventors identified that when a number of commercially relevant cultures where stored approximately at -46°C for 7 days or longer the individual pellets were sticking together and making larger clumps. In the industrial setting the clumping create handling problems. It is e.g. significantly more difficult to administer an adequate dose from the culture package when the culture is clumped. It may even be difficult to get the clumped culture out of the package in a convenient way.

Further studies identified that the "problematic" cultures could be characterized by having a Tm' value (onset of ice melting, as defined by Roos (1995)) of the pellet-frozen lactic acid bacteria (LAB) culture below the storage temperature of approximately -46°C. The Tm' value is a standard physical chemistry term used in the food industry and elsewhere. Tm' is routinely measured by Differential Scanning Caloriometry (DSC) techniques as described by Roos (1991). It relates to the onset temperature of melting of the food product (here the frozen LAB culture). For further details reference are made to the textbooks "Food Chemistry" Fennema (1996) and "Phase Transition in Foods" Roos (1995).

Without being limited to theory, it is believed that when a frozen culture has a Tm' value below its storage temperature , e.g. approximately -46°C, an initial phase transition (melting) occur and cause the individual pellets to stick together and form larger clumps.

In summary, the work of the present inventors has identified hitherto unrecognized storage problems in relation to the physical appearance of some types of commercially relevant highly concentrated pellet-frozen lactic acid bacteria cultures. Once having identified this problem, the present inventors could start to trying solve the problem.

Independent of any possible theoretical explanation, the present inventors identified that by adding certain relevant additive compounds to a problematic pellet-frozen culture, one could obtain a pellet-frozen culture, which after 7 to 14 days of storage at -46°C did not form clumps of individual pellets. Such cultures were characterized by that the individual pellets of the frozen culture were not sticking together and therefore substantially remain as individual pellets even after prolonged storage at approximately -46°C.

Overall, the relevant additive compounds may be characterized by that they are able to increase the Tm' value, of the frozen culture, to a value above the storage temperature, e.g. -46°C, such as for instance to raise the Tm' value from range-70 to -46°C to the range from -45 to -15°C.

The working examples herein describe preferred examples of suitable additive compounds. Described compounds include trehalose and maltodextrin.

As said above, in order to comprise sufficient bacteria a commercially relevant highly concentrated frozen culture generally has a weight of at least 50 g and a content of viable bacteria of at least 10⁹ colony forming units (CPU) per g. The cultures described in the articles of Chavarri (1988) and Carcoba (2000) are not directed to the physical stability of pellet-frozen cultures, but rather to the viability of the frozen bacteria in the present context not considered commercially relevant highly concentrated frozen cultures since they are made on much smaller scale and comprise significantly less grams of frozen culture, and moreover the described cultures are not pellet-frozen cultures. Also, the cultures described by Chavarri (1988) and Carcoba (2000) are not directed to the physical stability of pellet-frozen cultures al all, but rather to the viability of the frozen bacteria.

Accordingly, a first aspect of the invention relates to a pellet-frozen lactic acid bacteria (LAB) culture wherein the LAB is a Lactococcus spp. including Lactococcus lactis subsp. lactis and Lactococcus lactis subsp. cremoris in a commercially relevant package that has a weight of at least 50g frozen material, wherein the frozen material is present in the form of individual pellets, having a content of viable bacteria of at least 10⁹ colony forming units (CFU) per g frozen material and comprising from 0.5% to 13% of an additive compound measured as w/w of the frozen material, wherein the additive compound is an additive compound that is selected from the group of additive compounds consisting of Trehalose and Maltodextrine, and which further is characterized by,
when using an amount of 10% of the additive compound measured as w/w of the frozen material, the compound is able to increase the Tm' (onset temperature of ice melting) of the frozen lactic acid bacteria (LAB) culture, which without the additive compound has a Tm' value from -70°C to -46°C, to a Tm' value above -46°C, such as from -45°C to -15°C (measured by DSC)
and wherein the frozen lactic acid bacteria (LAB) culture is characterized by that when stored at approximately -46°C for 7-14 days the individual pellets of the frozen culture are not sticking together and therefore substantially remain as individual pellets where this is measured by following test
the individual pellets of the frozen culture are pellet frozen in liquid nitrogen and 100 individual pellets (around 5 - 100 g of pellets) are poured into a petridish, thus forming a thin layer of loose individual single pellets, the layer being characterized in that the majority of the pellets are in physically contact with one or more, of its neighbor pellets, placed at approximately - 46°C for 7-14 days and examined to see if the pellets are still loose or if the pellets had made clumps or are sticking together wherein the criteria for that the individual pellets of the frozen culture substantially remain as individual pellets are that at least 80 of the 100 individual pellets remain as loose individual single pellets;
with the exception of a frozen lactic acid bacteria (LAB) culture that comprises LAB that are able to utilize sucrose.

The "disclaimer" described at the end of the first aspect relates to the PCT application WO 2004/065584 A1. This application was filed 19 of January 2004. At the filing date of the priority forming application of the present application the PCT application WO 2004/065584 A1 was not published.

WO 2004/065584 A1 relates to improving the viability during storage of a frozen culture. It does not describe the "pellet sticking" problem of the present invention. The general, main claim 1 relates to "a frozen lactic acid bacteria (LAB) culture that comprises LAB that are able to utilize sucrose, has a weight of at least 50 g frozen material and a content of viable bacteria of at least 10⁹ colony forming units (CFU) per g frozen material, **characterized in that** the frozen culture comprises from 0.5% to 80% of a cryoprotective agent measured as w/w of the frozen material."

Although pellet-frozen cultures with cryoprotective agents are described in WO 2004/065584 A1, it can be excluded that a skilled person would inevitably arrive at a result falling within the terms of WO 2004/065584 A1 since WO 2004/065584 A1 specifically only claims cultures that are able to utilize sucrose.

With respect to the frozen culture of the present invention, the additive compound should preferably be added to the viable bacteria before they are frozen.

Accordingly, in a second aspect the invention relates to a method for making a pellet-frozen lactic acid bacteria (LAB) culture of the first aspect of the invention comprising following steps:
(i) adding an additive compound to viable bacteria to get at least 50 g of material with a content of viable bacteria of at least 10⁹ colony forming units (CPU) per g material and comprising the additive compound in an amount from 0.5% to 13% measured as w/w of the material,
(ii) freezing the material to get pellet-frozen material, and
(iii) packing the frozen material in a suitable way to get a packed pellet-frozen lactic acid bacteria (LAB) culture of the first aspect of the invention

A fourth aspect of the invention relates to use of the pellet-frozen lactic acid bacteria (LAB) culture as described above in a process for making a food or feed product.

### DEFINITIONS:

Prior to a discussion of the detailed embodiments of the invention a definition of specific terms related to the main aspects of the invention is provided.

The term "LAB that are able to utilize sucrose" denotes LAB that are able to ferment the sugar sucrose with the production of acids. This is the same definition as in PCT publication number WO 2004/065584 A1.

The term "material" of the culture denotes the relevant substances of the culture including both the viable bacteria and cryoprotective agent. Possible packing is not included. Consequently, the weight of the material of the culture is not including the weight of possible packing.

The term "packing" or "package" should be understood broadly. It denotes that the pellet-frozen lactic acid bacteria (LAB) culture should be packed in order to be provided to the user. It may be packed in a bottle, a tetra-pack^{©} container, etc.

The term "an additive compound" may in the present context be a single specific additive compound or it may be two or more different additive compounds. Accordingly, the w/w percentage of the additive compound(s) within the culture material should be understood as the sum of the amount of additive compound(s). Preferably, the term relates to a compound that is added to the culture after fermentation. Accordingly, it may be a compound that is not present in a significant amount in the culture fermentation broth as such.

The terms "pellet-frozen" and "pellet-frozen culture" refer to a culture frozen by use of a method which results in pellets or granula of the frozen culture. A pellet-frozen culture may conveniently be made by adding the culture dropwise into liquid N₂ forming frozen pellets or granula of the culture. Typically, but not necessarily, the process is performed on trays in a conventional industrial freeze-drying plant.

The term "pellets" or "granula" refers to small solid entities formed by frozen liquid of an average size between 0,1 and 10 mm.

### DRAWINGS

Figure 1: The correlation between the temperature at which the onset of ice melting occurs, Tm', and amount of disaccarides added can be seen in this figure. For further details, see working Example 3. The storage temperature, -46°C, is indicated by a punctured line.
Figure 2: Temperature of onset of ice melting (Tm') (Y-axis) of a number of cultures as a function of the maltodexdrin (Glucidex 12) concentration (% w/w) (X-axis). Culture name followed by "A" indicates that glycerine has been added to the culture, B indicates that glycerine was not added to the culture before pellet freezing.

### DETAILED DESCRIPTION OF THE INVENTION:

### Tm' value

As explained above, the Tm' value is a standard known term in physical chemistry describing the temperature at which the onset of ice melting occurs. In the present context Tm' denotes the temperature at which the onset melting of a frozen LAB culture occurs.

Preferably, the Tm' value is measured by use of the DSC protocol described in the section named "Measurement of Tm" of working example 1 herein.

### Pellet clumping test

As explained with respect to the first aspect of the invention the test to analyze if the pellet-frozen lactic acid bacteria (LAB) culture is a culture that may be characterized by that when stored at approximately -46°C (in the present situation a freezer preset to -50°C had an sample tempereture of -46°C) for 7-14 days the individual pellets of the frozen culture are not sticking together and therefore substantially remain as individual pellets in a test comprising following:
The individual pellets of the frozen culture are pellet frozen in liquid nitrogen and 100 individual pellets (around 5 - 100 g of pellets) are poured into a petridish, thus forming a thin layer of loose individual single pellets, the layer being characterized in that the majority of the pellets are in physically contact with one or more of its neighbor pellets, placed at approximately -46°C for 7-14 days and examined to see if the pellets are still loose or if the pellets had made clumps or are sticking together, wherein the criteria for that the individual pellets of the frozen culture substantially remain as individual pellets are that at least 80 of the 100 individual pellets remain as loose individual single pellets. More preferably at least 90 of the 100 individual pellets remain as loose individual single pellets and even more preferably at least 95 of the 100 individual pellets remain as loose individual single pellets.

Examining and counting individual pellets that remain as loose individual single pellets may be done visually. It is within the skilled person capacity to do this in a consistent way where the results would, within normal limited technical uncertainty, be consistent and repeatable. Working example 1 herein provides further technical details.

### A pellet-frozen lactic acid bacteria (LAB) culture

Preferably, the term "a frozen lactic acid bacteria (LAB) culture" denotes herein a culture which without comprising the added additive compound as described herein has a Tm' value of from -70 to -46°C. The culture may be frozen in the form of pellets or granula, forming a "a pellet-frozen lactic acid bacteria (LAB) culture". A pellet-frozen lactic acid bacteria (LAB) culture may conveniently be made by adding the culture dropwise into liquid N₂ forming frozen pellets or granula of the culture.

The LAB of the culture is a Lactococcus spp. that does not utilize sucrose according to International IDF Standard 146A:1998 "Identification of Characteristic Microorganisms" by use of apropriate API test kits (bioMérieux SA, Lyon, France). API kit "rapid ID 32 STREP" and "50 CHL Medium" is used to establish the sucrose utilization status for most LAB genera.

The LAB is a *Lactococcus* spp. including *Lactococcus lactis* subsp. *lactis* and *Lactococcus lactis* subsp. *cremoris.*

Even though some of these species in general are described as capable of utilizing sucrose mutants that are not able to utilize sucrose, have been, and will continuously be isolated. No matter how such mutations are isolated or obtained, they are still an aspect of the present invention.

The industrially most useful lactic acid bacteria are found among *Lactococcus* species, *Streptococcus* species, *Enterococcus* species, *Lactobacillus* species, *Leuconostoc* species and *Pediococcus* species.

The term "mixed lactic acid bacteria (LAB) culture" denotes a mixed culture that comprises two or more different LAB species. The term a "pure lactic acid bacteria (LAB) culture" denotes a pure culture that comprises only a single LAB species specie.

The culture as described herein may be a mesophilic culture consisting of mesophilic bacteria having optimum growth temperatures at about 30°C. A "a mesophilic culture" is a culture that comprises two or more different mesophilic LAB species.

An so-called O-culture is used to make cheese without holes (Cheddar, Cheshire, Feta) and typically comprises one or more organisms selected from the group comprising Lactococcus lactis subsp. lactis and Lactococcus lactis subsp. cremoris. In general O-cultures are considered not to utilize sucrose.

### Highly concentrated pellet-frozen lactic acid bacteria cultures

The frozen cultures as described herein are what in the food industry may be termed highly concentrated pelletfrozen lactic acid bacteria cultures. In order to comprise sufficient bacteria, such cultures should be relatively big (have a sufficient weight) combined with a relatively high concentration of viable bacteria. It is obvious that if relatively more bacteria is required, the weight and/or the concentration of viable bacteria should be increased.

Preferably, a pellet-frozen lactic acid bacteria (LAB) culture as described herein has a weight of at least 100 g frozen material, more preferably a weight of at least 250 g frozen material, even more preferably a weight of at least 500 g frozen material and most preferably a weight of at least 900 g frozen material. Preferably, the weight of the frozen material is less than 500 kg.

Preferably, a pellet-frozen lactic acid bacteria (LAB) culture as described herein has a content of viable bacteria of at least 5×10⁹ colony forming units (CFU) per g frozen material, more preferably a content of viable bacteria of at least 10¹⁰ colony forming units (CFU) per g frozen material, and most preferably a content of viable bacteria of at least 2×10¹⁰ colony forming units (CFU) per g frozen material.

Fermentation and suitable fermentation media for LAB are known in the art and the skilled person is capable of selecting a suitable media and fermentation conditions in relation to the specific LAB. Suitable media and fermentations are given in the working example section herein.

In order to get sufficient amount of bacteria, it is in the present context preferred to make a relatively large-scale fermentation in suitable big fermentation tanks. Fermentation tanks of at least 50 L, preferably at least 90 L, even more preferably 500 L or bigger are preferred.

After a suitable fermentation, the viable bacteria are preferably isolated by removal of the liquid (supernatant) of the fermentation media (e.g. by centrifugation). The isolated viable bacteria may be termed the isolated biomass. The isolated viable bacteria shall preferably have a content of viable bacteria of at least 10⁹ colony forming units (CFU) per g frozen material, more preferably a content of at least 5×10⁹ CFU per g frozen material, and most preferably a content of at least 10¹⁰ CFU per g frozen material.

After addition of the additive compound (see below) to the concentrated culture. The culture may conveniently be frozen by adding the mixture dropwise into liquid N₂ forming frozen pellets or granula of the mixture. A feasible method for the freezing process is described in DE2805676 and FR2393251.

The pellet-frozen culture is then packaged a suitable way in order to be provided to the user.

### Additive compound

As discussed above, preferably the relevant additive compounds are characterized by that they are able to increase the Tm' value, of the frozen culture, to a value above the storage temperature, e.g. -46°C, such as to a Tm' value from -45°C to -15°C, more preferably to a Tm' value from -43°C to -15°C and even more preferably to a Tm' value from -39°C to -15°C.

Working example 2 herein illustrates a rapid experimental strategy to identify relevant additive compounds. To a "model" frozen culture with a Tm' value below -46°C (in example 2 "model" culture has a Tm' value of -54°C) was added different relevant compounds (10% W/W) and the Tm' values before and after addition were measured by DSC.

The "model" culture of example 2 and the test protocol of this example 2 is preferably used to evaluate if specific additive compounds of interest may be characterized by that they are able to increase the Tm' value, of the frozen culture, to a value above the -46°C such as to a Tm' value from -45°C to -15°C, more preferably to a Tm' value from -43°C to -15°C, even more preferably to a Tm' value from -39°C to -15°C.

In working example 2 it can be seen that Cyclodextrin increased Tm' to -44°C, Maltitol increased Tm' to -42°C, Trehalose increased Tm' to -38°C, Fish gelatin increased Tm' to - 37°C, Maltodextrine increased Tm' to -32°C and Spray gum increased Tm' to -31°C.

The frozen culture comprises of 0,5% to 13% of an additive compound measured as w/w of the frozen material, preferably from 1% to 12% of an additive compound measured as w/w of the frozen material, more preferably from 2% to 10% of an additive compound measured as w/w of the frozen material and even more preferably from 5% to 10% of an additive compound measured as w/w of the frozen material.

The addition of the additive compound, which may also be a cryoprotective agent, after fermentation, to the isolated viable bacteria (biomass) may be done by mixing solid additive compound with the biomass for e.g. 30 minutes at a suitable temperature. If the additive compound agent is e.g. maltodextrin a suitable temperature may be room temperature. Alternatively, a sterile solution of the additive compound may be mixed with the biomass.

### A method for making a pellet-frozen lactic acid bacteria (LAB) culture

As said above, a second aspect of the invention relates to a method for making a pellet-frozen lactic acid bacteria (LAB) culture of the first aspect of the invention comprising following steps:
(i) adding an additive compound to viable bacteria to get at least 50 g of material with a content of viable bacteria of at least 10⁹ colony forming units (CFU) per g material and comprising the additive compound in an amount from 0.5% to 13% measured as w/w of the material,
(ii) freezing the material to get pellet-frozen material, and
(iii) packing the pellet-frozen material in a suitable way.

As discussed above, the herein most relevant "problematic" cultures are pellet-frozen lactic acid bacteria (LAB) cultures, which without comprising the added additive compound as described herein has a Tm' value of from -70°C to -46°C.

Accordingly, in a preferred embodiment before making the addition of the additive compound according to step (i) above one has measured the Tm' value of the frozen lactic acid bacteria (LAB) culture without comprising the additive compound and identified that it has a Tm' value of from -70°C to -46°C or even lower.

Before making the addition of the additive compound according to step (i) above one has performed a pellet clumping test (see above) and identified that the individual pellets of the frozen culture stick together at storage at -46°C.

Preferably, after the addition of the additive compound the Tm' value of the frozen lactic acid bacteria (LAB) culture comprising the additive compound is measured and it is verified that the Tm' value is above -46°C, preferably from -45°C to -15°C, more preferably from -43°C to -15°C and even more preferably from -39°C to -15°C.

Finally, after the addition of the additive compound the culture is pellet-frozen and a pellet clumping test (see above) ensuring that at least 80 of the 100 individual pellets remain as loose, individual single pellets, is performed.

### Use of the frozen lactic acid bacteria (LAB) culture

A frozen lactic acid bacteria (LAB) culture as described herein may be used in a process for making a food or feed product according to the art.

### EXAMPLES:

### Example 1:

R604-E (a commercially available frozen *O-culture*, Chr. Hansen A/S, Denmark) tends to form sticky pellets during frozen storage. In the present study this problem is approached by taking a closer look at the melting temperature, and trying to increase it by adding caseinate, sucrose or maltodextrin.

### Aim:

To evaluate if it is possible to raise the melting point of F-DVS of R604-E by using additives.

The effect of using additives to increase the melting point of R604-E is evaluated:
visually, and
by measuring the Tm' by DSC for each of the tested formulations.

### i) Material:

2 kilos of commercially available culture, F-DVS R 604-E (Chr. Hansen A/S, Hoersholm, Denmark, Batch 2441258, material no. 616581).

### ii) Additives solution used for formulation to raise the melting point:

50% (w/w) sucrose solution (Danisco, Denmark).
10% (w/w) Na-caseinate solution (Arla, Denmark).
30% (w/w) Malto Dextrin DE 10 solution (Glucidex 10, Roquette Frères, Lestrem, France).
30% (w/w) Malto Dextrin DE 2 solution (Glucidex 2, Roquette Frères, Lestrem, France).

### iii) Recipe for formulation of F-DVS R604-E:

The frozen concentrate was thawed and mixed with additives according to Table 1.

**Table 1. Formulations of R604-E**

| **Formulation ID** | **Amount cell concentrate (g)** | **Additives (g)** | **Dilution of conc. x** | **Additives* (%)** |
|---|---|---|---|---|
| F-DVS R604E/Reference | 300 | 0 | 1 | 0 |
| F-DVS R604E/6% Sucrose | 300 | 41 | 1,14 | 6 |
| F-DVS R604E/10% Sucrose | 300 | 75 | 1,25 | 10 |
| F-DVS R604E/6% Malto Dextrin DE 10 | 300 | 75 | 1,25 | 6 |
| F-DVS R604E/6% Malto Dextrin DE 2 | 300 | 75 | 1,25 | 6 |
| F-DVS R604E/2% Na-caseinate | 300 | 75 | 1,25 | 2 |
| *) g dry matter additive / g concentrate | | | | |

### Visual evaluation of melting point of F-DVS R604.

The 6 formulations of F-DVS R-604 E were pellet-frozen in liquid nitrogen and 100 individual pellets (around 20 - 30 g) of pellets were poured into petridishes, thus forming a thin layer of loose, single pellets.

One sample of each of the formulations was placed in a freezer preset to at -50°C, the actual temperature of the samples was -46°C. After 7 days of storage the samples were examined to see if they were still loose or if the pellets had made clumps or seemed sticky - and if so - their willingness to be shaken into loose particles again.

**Table 2; Visual inspection of frozen pellets and measured Tm'.**

| **Sample marked :** | **Stored at minus -46°C** | **Tm' measured in samples stored at -46°C, 6 days** |
|---|---|---|
| F-DVS R604-E Batch 2441258 Reference | - | -56 |
| F-DVS R604-E Batch 2441258 6% Sucrose | - | -49 |
| F-DVS R604-E Batch 2441258 10% Sucrose | ++ | -42 |
| F-DVS R604-E Batch 2441258 6% Malto Dextrin DE 10 | +++ | -44 |
| F-DVS R604-E Batch 2441258 6% Malto Dextrin DE 2 | +++ | -42 |
| F-DVS R604-E Batch 2441258 2% Na-caseinate | - | -58 |
| - = Clump, caked or sticky. (less than 20 of the 100 individual pellets remain as loose individual single pellets) | | |
| + = Partly loose particles. (less than 60 of the 100 individual pellets remain as loose individual single pellets) | | |
| ++ = Nearly loose particles. (at least 80 of the 100 individual pellets remain as loose individual single pellets) | | |
| +++ = Loose particles (at least 90 of the 100 individual pellets remain as loose individual single pellets) | | |

### Measurement of Tm':

The samples were prepared in 100 µL alumina crucibles and frozen in liquid nitrogen. One sample of each of the formulations and F-DVS R604 was placed for 6 days at -46°C.

The phase transition was measured on a Mettler Toledo 822e Differential Scanning Caloriometer (a DSC) with 100 µL alumina crucibles, temperature program, insert temp -90°C, scanning temp. program: 5°C/min. -130°C - 0°C.

The Tm' values (onset of ice melting, as defined by Roos (1995)) were measured. Results are shown in table 2.

We observe that use of >6% sucrose and 6% malto dextrin (2 or 10) increase the Tm' value of frozen pellets. It is not possible to see any effect of Na-caseinate. From the visual inspection 10% sucrose and the two different maltodextrin show positive effect against the tendency to make sticky pellets.

### Example 2:

### Aim

To identify what kind of additives that could increase the melting point of frozen culture a screening study was made. The following additives were tested:
Trehalose, Malto dextrin 12, cyclo dextrin, spray gum, PEG, Fish gelatin, maltitol, Sodium chloride, glycerol.

### i) Material:

F-DVS R 604-E (Batch 2471755, material 616581) for details please see Example1.

### ii) Additives solution used for formulation to raise the melting point:

50% (w/w) trehalose.
30% (w/w) Malto Dextrin DE 12 (Glucidex 12, Roquette Frères, Lestrem, France)..
30% (w/w) cyclodextrin
30% (w/w) Spray gum (IRX 51693 from CNI)
30% (w/w) PEG (PEG 6000, Merck, Germany)
30% (w/w) Fish gelatin bloom 200 (SKW Biosystems, France)
30% (w/w) maltitol
30% (w/w). Sodium Chloride
30% (w/w) Glycerol

### iii) Recipe for formulation of F-DVS R604-E:

The frozen F-DVS R604-E concentrate was thawed and mixed with the different additives to a final formulation of 10% (W/W).

### Measurement of Tm':

The samples were prepared in 100 µL alumina crucibles and frozen in liquid nitrogen. The phase transition curves were recorded on the Mettler Toledo 822e Differential Scanning Caloriometer for the 9 formulations and compared to the reference sample (R604E without additives). The samples were inserted to the DSC at -90°C and run using temperature program: insert temperature -90°C; temperature scanning 7°C /min from -130°C to 0°C.

### Result:

The phase transition curves were made and the Tm' values determined as described by Roos (1991) the values are given in table 3 below:

**Table 3; Observed Tm' (°C).**

| **Additive** | **Tm' (°C)** |
|---|---|
| Trehalose | -38 |
| Malto Dextrin DE 12 | -32 |
| Cyclodextrin | -44 |
| Spray gum (IRX 51693) | -31 |
| PEG 6000 | -52 |
| Fish gelatin bloom 200 | -37 |
| Maltitol | -42 |
| Sodium Chloride | -62 |
| Glycerol | -54 |
| Culture, R-604 E | -54 |

The Tm' for the reference sample is found to be -54°C (onset of ice melting).

The following additives are increasing Tm':
PEG (-53°C),
Cyclodextrin (-44°C),
Maltitol (-42°C),
Trehalose (-38°C)
Fish gelatin (-37°C)
Maltodextrine 12 (-32°C)
Spray gum (-31 °C)

Glycerol and sodium chloride did not increase the melting point of frozen culture pellets.

### Reference

### Example 3:

In this trial the intension was to evaluate the relationship between amount of additive and the increase in Tm' measured on DSC.

### i) Material:

F-DVS CH N 19 (Batch 2421868) (commercially available frozen *LD-cultures,* Chr. Hansen A/S, Denmark).

**Table 4. Formulation CH N19 using sucrose and trehalose as additive agents.**

| Formulation | cell concentrate | 50 % Sucrose | Dilution of conc | Sucrose |
|---|---|---|---|---|
| ID | (g) | (g) | X | % |
| F-DVS/-additives (Reference) | 300 | 0,00 | 1,00 | 0,00 |
| F-DVS/5% Trehalose | 300 | 43,00 | 1,14 | 4,99 |
| F-DVS/3% Sucrose | 300 | 19,00 | 1,06 | 2,98 |
| F-DVS/5% Sucrose | 300 | 34,00 | 1,11 | 5,09 |
| F-DVS/6% Sucrose | 300 | 42,00 | 1,14 | 6,14 |
| F-DVS/8% Sucrose | 300 | 57,00 | 1,19 | 7,98 |
| F-DVS/9% Sucrose | 300 | 66,00 | 1,22 | 9,02 |
| F-DVS/10% Sucrose | 300 | 75,00 | 1,25 | 10,00 |
| F-DVS/13% Sucrose | 300 | 105,00 | 1,35 | 12,96 |

### ii) Additive solutions used for formulation to raise the melting point:

The concentration of sucrose per gram biomass varied from 3% (w/w) up to 13% (w/w). Trehalose was only tested on a 5% (w/w) level. All sucrose concentrations were prepared from a 50% (w/w) sucrose solution added to the biomass. The trehalose concentration was prepared from a 40% (w/w) solution.

### Measurement of Tm':

The frozen F-DVS R604-E concentrate was thawed and mixed with the different additives as indicated in table 4. Then samples were transferred to 100 µL alumina crucibles and frozen in liquid nitrogen and stored at -46°C before analyzed. The phase transition curves were recorded on the DSC for the 8 formulations and compared to the reference sample (R604E without additives). The samples were inserted into the DSC at -90°C and run using temperature program: insert temperature -90°C; temperature scanning: 5°C/min. -130°C - 0°C.

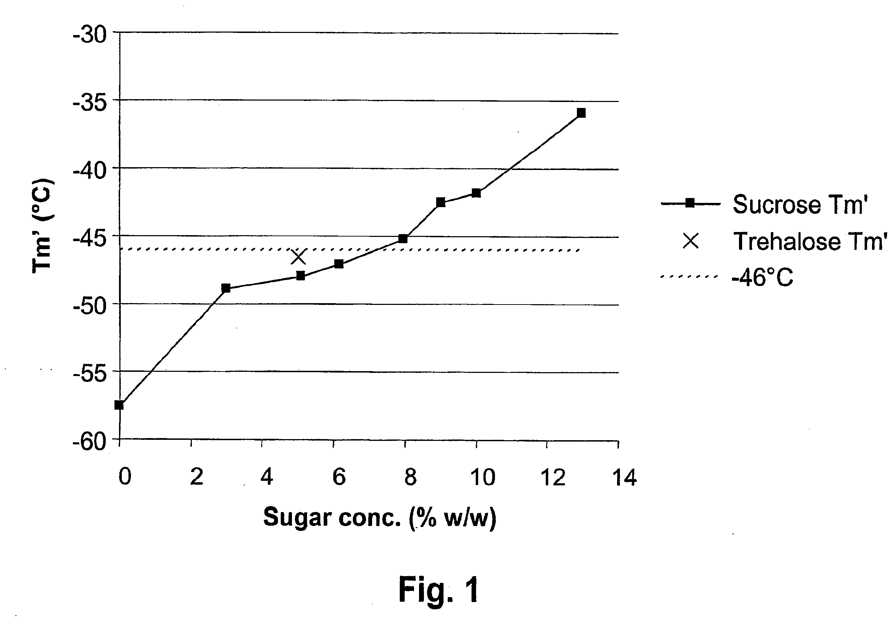

From these phase transition curves the Tm'. The correlation between Tm' and amount of disaccarides added can be seen in Figure 1.

From Figure 1 it can be seen that 8% sucrose and more will ensure that the frozen culture do not start melting at -46°C storage.

### Example 4

Commercially available cultures from Chr. Hansen A/S, Denmark (HP, HPS, HP-1, LP, LL-2) were analyzed for initial melting point before and after addition of maltodextrin (Glucidex 12 from Roquette Frères, Lestrem, France). The products are sold as frozen pellets, and these should be kept loose which is secured by an initial melting point above the storage temperature.

### Aim:

The aim of the present study was to raise the initial melting point above storage temperature in order to obtain loose pellets.

### Materials and methods:

### i) Materials

Glucidex 12 (Roquette Frères, Lestrem, France) was used as additive compound.

100 g of each of the cultures listed in Table 5 was used. B indicates that glycerine was not added to the culture, where as A indicates that 10% v/v glycerine has been added before pellet freezing.

**Table 5: Cultures used**

| **Material no.** | **Product** | **Batch no.** |
|---|---|---|
| 73258 | HP A/B | 2511924 |
| 73264 | HPS A | 2511063 |
| 73270 | HP-1 A | 2511070 |
| 72045 | LP A/B | 2511919 |
| 71468 | LL-2 A/B | 2513227 |

### ii) Recipe for formulation of the samples:

Frozen concentrate cultures were thawed and mixed with the different amounts of a Glucidex 12 solution to a final formulation of 3.5% to 10.1% (W/W).

The different formulations are listed in Table 6.

### iii) Measurement of Tm':

The samples were prepared in 100 µL alumina crucibles. The phase transition curves were recorded on the Mettlet DSC for all the formulations. The samples were inserted to the DSC at - 90°C. Scanning temperature program: 7°C/min. -100°C - 0°C.

### iiii) Visual evaluation of melting point of F-DVS R604.

The formulations were pellet-frozen in liquid nitrogen and 100 individual pellets (around 20 - 30 g) of pellets were poured into petridishes, thus forming a thin layer of loose single pellets. One sample of each of the formulations was placed at -46°C. After 14 days of storage the samples were examined to see if they were still loose or if the pellets had made clumps or seemed sticky - and if so - their willingness to be shaken into loose particles again.

### Results:

Results of adding maltodextrine (Glucidex 12) to different cultures in various amounts are shown in Figure 1. It is clear that increasing the concentration of maltodextrine increases Tm'. In Table 2 the results on evaluating the stickiness/clumping are listed. Samples with a Tm' above the storage temperature of -46° were loose pellets where as the samples having Tm' below -46°C were sticking together.

**Table 6: Formulations of samples, Initial melting temperature (Tm') and visual inspection of frozen pellets.**

| **Sample** | **Maltodextrin %** | **Tm'** | **Stored at minus 46°C** |
|---|---|---|---|
| HP B | 4.0 | -46 | **-** |
| HP B | 6.6 | -40 | **+++** |
| HP B | 10.1 | -35 | **+++** |
| HP A | 3.5 | -56 | **-** |
| HP A | 6.5 | -53 | **-** |
| HP A | 10.0 | -49 | **-** |
| HP-1 A | 6.0 | -57 | **-** |
| HPS A | 0.0 | -58 | **not measured** |
| HPS A | 6.1 | -53 | **-** |
| LP B | 6.0 | -45 | **+++** |
| LP A | 0.0 | -60 | **not measured** |
| LP A | 6.0 | -54 | **-** |
| LL-2 B | 6.1 | -42 | **+++** |
| LL-2 A | 0.0 | -61 | **not measured** |
| LL-2 A | 6.1 | -54 | **-** |
| Note to table: | | | |
| - designates clumped, caked or sticky (less than 20 of the 100 individual pellets remain as loose individual single pellets). | | | |
| +++ designates Loose particles (at least 90 of the 100 individual pellets remain as loose individual single pellets). | | | |

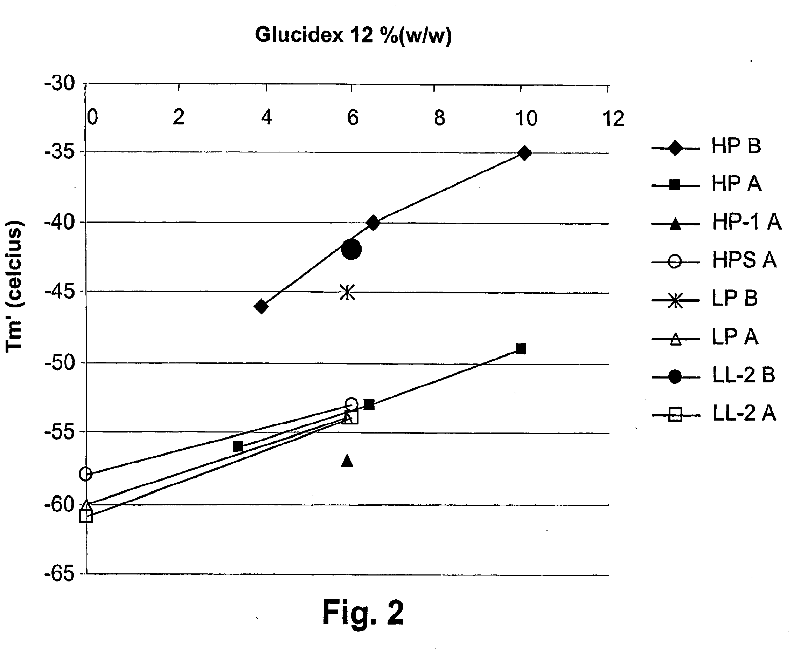

The result is illustrated in table 6 and in fig. 2 and shows that maltodextrin is an effective agent to increase the Tm' in cultures containing 10% v/v glycerine (A-series) as well in cultures not containing glycerine (B-series). This experiment further demonstrates that a well known cryoprotectant (i.e. glycerine) not can be used to increase the physical stability during storage of a pellet-frozen culture.

### REFERENCES:

Cárcoba, R. et al., "Influence of cryoprotectants on the viability and acidifying activity of frozen and freeze-dried cells of the novel starter strain Lactococcus lactis subsp. lactis CECT 5180", Eur Food Res Technol (2000) 211, 433 - 437
Chavarri, F.J. et al, "Cryoprotective agents for frozen concentrated starters from non-bitter Streptococcus Lactis strains", Biotechnology letters, vol 10, 1, 11- 16 (1988*)*
DE2805676, (Jespersen et al.), 18 August 1978.
Fennema O. R. (ed.) Food Chemistry, 3. ed. Marcel Dekker, 1067 pp. (ISBN: 0-8247-9346-3), 1996.
FR2393251, (Jespersen et al.), 29 December 1978.
IDF (1998), International IDF Standard 146A:1998 "Identification of Characteristic Microorganisms".
Roos, Y. and M. Karel (1991) Phase transitions of amorphous sucrose and frozen sucrose solutions. J. Food Science, 56:266-267.
ROOS, Y. Phase Transition in Foods. ACADEMIC PRESS, New York, USA. 360 pp., (ISBN: 0-12-595340-2), 1995.
US 4,140,800 (Leo Kline) 20 February 1979.
WO 00/39281 (Kringelum et al.) 6 July 2000.
WO 2004/065584 A1 (Bisgaard-Frantzen et al.) 5 August 2004 (similar to International Application Number PCT/DK2004/000025)

## Claims

1. A pellet-frozen lactic acid bacteria (LAB) culture wherein the LAB is a Lactococcus spp. including Lactococcus lactis subsp. lactis and Lactococcus lactis subsp. cremoris in a commercially relevant package that has a weight of at least 50g frozen material, wherein the frozen material is present in the form of individual pellets, having a content of viable bacteria of at least 10⁹ colony forming units (CFU) per g frozen material and comprising from 0.5% to 13% of an additive compound measured as w/w of the frozen material, wherein the additive compound is an additive compound that is selected from the group of additive compounds consisting of Trehalose and Maltodextrine, and which further is **characterized by,**
when using an amount of 10% of the additive compound measured as w/w of the frozen material, the compound is able to increase the Tm' (onset temperature of ice melting) of the frozen lactic acid bacteria (LAB) culture, which
without the additive compound has a Tm' value from -70°C to -46°C, to a Tm' value above -46°C, such as from -45°C to -15°C (measured by DSC)
and wherein the frozen lactic acid bacteria (LAB) culture is **characterized by** that when stored at approximately -46°C for 7-14 days the individual pellets of the frozen culture are not sticking together and therefore substantially remain as individual pellets where this is measured by following test
the individual pellets of the frozen culture are pellet frozen in liquid nitrogen and 100 individual pellets (around 5 - 100 g of pellets) are poured into a petridish, thus forming a thin layer of loose individual single pellets, the layer being **characterized in that** the majority of the pellets are in physically contact with one or more, of its neighbor pellets, placed at approximately -46°C for 7-14 days and examined to see if the pellets are still loose or if the pellets had made clumps or are sticking together wherein the criteria for that the individual pellets of the frozen culture substantially remain as individual pellets are that at least 80 of the 100 individual pellets remain as loose individual single pellets;
with the exception of a frozen lactic acid bacteria (LAB) culture that comprises LAB that are able to utilize sucrose.

2. The pellet-frozen culture of claim 1, wherein the frozen lactic acid bacteria (LAB) culture is a culture which without comprising the additive compound according to claim 1 has a Tm' value of from -70°C to -46°C.

3. The pellet-frozen culture of any of the preceding claims, wherein the frozen lactic acid bacteria culture comprises from 5% to 10% of the additive compound measured as w/w of the frozen material.

4. A method for making a pellet-frozen lactic acid bacteria (LAB) culture of any of the claims 1 to 3 comprising the following steps:
(i) adding an additive compound to viable bacteria to get at least 50 g of material with a content of viable bacteria of at least 10⁹ colony forming units (CFU) per g material and comprising the additive compound in an amount from 0.5% to 13% measured as w/w of the material,
(ii) freezing the material to get pellet-frozen material, and
(iii) packing the pellet-frozen material in a suitable way to get a packed frozen lactic acid bacteria (LAB) culture of any of the claims 1 to 3.

5. The method of claim 4, wherein
before adding the additive compound according to step (i) of claim 4 one has measured the Tm' value of the frozen lactic acid bacteria (LAB) culture without comprising the additive compound and identified that it has a Tm' value of from - 70°C to -46°C;
and
after adding the additive compound is the Tm' value of the frozen lactic acid bacteria (LAB) culture comprising the additive compound measured and it is verified that the Tm' value is from -49°C to -15°C, more preferably from -39°C to -15°C.

6. The method of claim 4 to 5, wherein the frozen lactic acid bacteria culture comprises from 5% to 10% of the additive compound measured as w/w of the frozen material.

7. Use of the pellet-frozen lactic acid bacteria (LAB) culture of any of claims 1-3 in a process for making a food or feed product.

## Patentansprüche

1. In Pellets gefrorene Kultur aus Milchsäurebakterien (LAB - lactic acid bacteria), wobei die LAB eine *Lactococcus* spp. Kultur ist, darunter *Lactococcus lactis* subsp. *lactis* und *Lactococcus lactis* subsp. *cremoris,* in einer kommerziell relevanten Packung, die ein Gewicht von mindestens 50g gefrorener Materie aufweist, wobei die gefrorene Materie in Form von einzelnen Pellets vorliegt, mit einem Gehalt an lebensfähigen Bakterien von mindestens 10⁹ Kolonien-bildenden Einheiten (CFU - colony forming units) pro g gefrorener Materie und mit 0.5% bis 13 % Gewicht/Gewicht der gefrorenen Materie eines Zusatzstoffes, wobei die der Zusatzstoff aus der Gruppe von Zusatzstoffen ausgewählt wurde, die aus Trehalose und Maltodextrin besteht, und die Kultur ferner **dadurch gekennzeichnet ist, dass**
bei Benutzung von einer Menge von 10% Gewicht/Gewicht der gefrorenen Materie des Zusatzstoffs, die Verbindung die Schmelztemperatur Tm (Temperatur, bei der das Schmelzen einsetzt) der gefrorenen Kultur der Milchsäurebakterien (LAB) erhöhen kann, welche ohne Zusatzstoffe einen Tm-Wert von -70°C bis -46°C aufweist, bis auf einen Tm-Wert von über -46°C, wie zum Beispiel -45°C bis -15°C (gemessen durch dynamische Differenzkalorimetrie, DSC)
und wobei die gefrorene Kultur aus Milchsäurebakterien (LAB) **dadurch gekennzeichnet ist, dass,** wenn sie 7 bis 14 Tage lang bei ungefähr -46°C gelagert wird, die einzelnen Pellets der gefrorenen Kultur nicht zusammenkleben und daher im wesentlichen als einzelne Pellets verbleiben, wobei dies durch folgenden Test gemessen wird, bei dem die einzelnen Pellets der gefrorenen Kultur in flüssigem Stickstoff in Pellets eingefroren werden und 100 einzelne Pellets (ca. 5-100 g Pellets) in eine Petrischale geschüttet werden, wo sie somit eine dünne Schicht loser einzelner Pellets bilden, wobei die Schicht **dadurch gekennzeichnet ist, dass** die meisten Pellets mit einem oder mehreren seiner Nachbarpellets in Kontakt sind, die 7 bis 14 Tage lang bei ungefähr -46°C belassen werden und überprüft wird, um zu ermitteln, ob die Pellets noch lose sind oder ob die Pellets Klumpen gebildet haben oder zusammenkleben, wobei mindestens 80 der 100 einzelnen Pellets als einzelne Pellets der gefrorenen Kultur verbleiben;
wobei eine gefrorenen Kultur aus Milchsäurebakterien (LAB) ausgenommen ist, welche Milchsäurebakterien LAB umfasst, die in der Lage sind, Saccharose zu verwenden.

2. In Pellets gefrorene Kultur gemäß Anspruch 1, wobei die gefrorene Kultur aus Milchsäurebakterien (LAB) eine Kultur ist, welche ohne den Zusatzstoff gemäß Anspruch 1 einen Tm-Wert von -70°C bis -46°C aufweist.

3. In Pellets gefrorene Kultur gemäß einem der vorherigen Ansprüche, wobei die gefrorene Kultur aus Milchsäurebakterien 5% bis 10% Gewicht/Gewicht der gefrorenen Materie des Zusatzstoffes umfasst.

4. Verfahren zur Herstellung einer in Pellets gefrorenen Kultur aus Milchsäurebakterien (LAB) gemäß einem der Ansprüche 1 bis 3, das Schritte umfasst, bei denen man:
(i) ein Zusatzmittel zu lebensfähigen Bakterien zugibt, um mindestens 50g einer Materie mit einem Gehalt an lebensfähigen Bakterien von mindestens 10⁹ Kolonien-bildenden Einheiten (CFU) pro g der Materie und mit dem Zusatzstof in einer Menge von 0.5% bis 13% Gewicht/Gewicht zu erhalten,
(ii) die Materie einfriert, um eine in Pellets gefrorene Materie zu erhalten, und
(iii) die in Pellets gefrorene Materie auf zweckmäßige Weise verpackt, um verpackte gefrorene Kultur aus Milchsäurebakterien (LAB) gemäß einem der Ansprüche 1 bis 3 zu erhalten.

5. Verfahren gemäß Anspruch 4, bei dem man
vor dem Hinzufügen des Zusatzstoffes in Schritt (i) des Anspruchs 4 den Tm-Wert der gefrorenen Kultur aus Milchsäurebakterien (LAB) ohne den Zusatzstoff bestimmt, wobei dieser einen Tm-Wert von -70°C bis -46°C aufweist; und
nach dem Hinzufügen des Zusatzstoffes den Tm-Wert der gefrorenen Kultur aus Milchsäurebakterien (LAB) mit dem Zusatzstoff bestimmt und bestätigt, dass der Tm-Wert im Bereich von -49°C bis -15°C liegt, besonders bevorzugt im Bereich von -39°C bis -15°C.

6. Verfahren gemäß Anspruch 4 oder 5, bei dem die gefrorene Kultur aus Milchsäurebakterien 5% bis 10% Gewicht/Gewicht der gefrorenen Materie des Zusatzstoffes umfasst.

7. Verwendung der in Pellets gefrorenen Kultur aus Milchsäurebakterien (LAB) gemäß einem der Ansprüche 1 bis 3 in einem Verfahren zur Herstellung eines Lebensmittels oder Futters.

## Revendications

1. Culture de bactérie lactique (BL) congelée en pastilles, laquelle BL est une espèce Lactococcus, y compris les sous-espèces Lactococcus lactis lactis et Lactococcus lactis cremoris, dans un emballage approprié du point de vue commercial et contenant un poids de matière congelée d'au moins 50 g et laquelle matière congelée se trouve sous forme de pastilles individuelles, présentant une teneur en bactéries viables d'au moins 10⁹ unités formant colonie (UFC) par gramme de matière congelée et contenant de 0,5 à 13 % d'un adjuvant, en pds/pds de matière congelée, lequel adjuvant est un adjuvant choisi dans l'ensemble d'adjuvants formé par le tréhalose et la maltodextrine, et qui est en outre **caractérisée en ce que,**
lorsqu'on utilise l'adjuvant en une proportion de 10 % en pds/pds de matière congelée, cet adjuvant peut augmenter la Tm' (température de début de fusion de la glace) de la culture de bactérie lactique (BL) congelée, laquelle, sans l'adjuvant, présente une Tm' qui vaut de ―70 à ―46 °C, jusqu'à une valeur de Tm' supérieure à ―46 °C, telle que de ―45 à ―15 °C (mesurée par AED),
et laquelle culture de bactérie lactique (BL) congelée est **caractérisée en ce que,** lorsqu'elle est conservée à environ ―46 °C durant 7 à 14 jours, les pastilles individuelles de la culture congelée n'adhèrent pas les unes aux autres et restent donc pratiquement à l'état de pastilles individuelles, ceci étant mesuré par le test suivant :
― les pastilles individuelles de la culture congelée étant des pastilles congelées dans de l'azote liquide, 100 pastilles individuelles (soit environ 5 à 100 g de pastilles) sont versées dans une boîte de Petri où elles forment une fine couche de simples pastilles individuelles libres, laquelle couche est **caractérisée en ce que** la plupart des pastilles sont en contact physique avec une ou plusieurs des pastilles voisines, puis on les laisse à environ ―46 °C durant 7 à 14 jours et on les examine afin de voir si les pastilles sont encore libres ou si les pastilles ont formé des amas ou sont collées les unes aux autres, étant entendu que le critère pour que les pastilles individuelles de la culture congelée restent pratiquement à l'état de pastilles individuelles est qu'au moins 80 des 100 pastilles individuelles restent à l'état de simples pastilles individuelles libres;
à l'exception d'une culture de bactérie lactique (BL) congelée qui comprend des BL capables d'utiliser du saccharose.

2. Culture congelée en pastilles conforme à la revendication 1, laquelle culture de bactérie lactique (BL) congelée est une culture qui, en l'absence de l'adjuvant indiqué dans la revendication 1, présente une température Tm' valant de ―70 à ―46 °C.

3. Culture congelée en pastilles conforme à l'une des revendications précédentes, laquelle culture de bactérie lactique congelée contient de 5 à 10 % de l'adjuvant, en pds/pds de matière congelée.

4. Procédé de préparation d'une culture de bactérie lactique (BL) congelée en pastilles, conforme à l'une des revendications 1 à 3, comprenant les étapes suivantes :
(i) ajouter un adjuvant à des bactéries viables, afin d'obtenir au moins 50 g de matière présentant une teneur en bactéries viables d'au moins 10⁹ unités formant colonie (UFC) par gramme de matière et contenant l'adjuvant en une proportion de 0,5 à 13 %, en pds/pds de matière,
(ii) congeler la matière afin d'obtenir une matière congelée en pastilles, et
(iii) emballer la matière congelée en pastilles de manière adéquate afin d'obtenir une culture de bactérie lactique (BL) congelée emballée, conforme à l'une des revendications 1 à 3.

5. Procédé conforme à la revendication 4, dans lequel
― avant d'ajouter l'adjuvant conformément à l'étape (i) de la revendication 4, on a mesuré la température Tm' de la culture de bactérie lactique (BL) congelée ne contenant pas l'adjuvant et vérifié que celle-ci présente une Tm' valant de ―70 à ―46 °C ;
― et après avoir ajouté l'adjuvant, on mesure la température Tm' de la culture de bactérie lactique (BL) congelée contenant l'adjuvant, et l'on vérifie que cette Tm' vaut de ―49 à ―15 °C, et de préférence de ―39 à ―15 °C.

6. Procédé conforme à la revendication 4 ou 5, dans lequel la culture de bactérie lactique congelée contient de 5 à 10 % de l'adjuvant, en pds/pds de matière congelée.

7. Utilisation d'une culture de bactérie lactique (BL) congelée en pastilles, conforme à l'une des revendications 1 à 3, dans un procédé de fabrication d'un produit alimentaire ou d'une nourriture pour animaux.
